(19) Europäisches Patentamt / European Patent Office / Office européen des brevets

(11) **EP 2 581 110 B1**

(12) **EUROPEAN PATENT SPECIFICATION**

(45) Date of publication and mention
of the grant of the patent:
**01.07.2015 Bulletin 2015/27**

(51) Int Cl.:
**A61N 5/10** (2006.01)

(21) Application number: **12193833.6**

(22) Date of filing: **30.11.2007**

(54) **Inner gantry**

Innerer Kran

Portique interne

(84) Designated Contracting States:
**AT BE BG CH CY CZ DE DK EE ES FI FR GB GR
HU IE IS IT LI LT LU LV MC MT NL PL PT RO SE
SI SK TR**

(43) Date of publication of application:
**17.04.2013 Bulletin 2013/16**

(62) Document number(s) of the earlier application(s) in
accordance with Art. 76 EPC:
**11165422.4 / 2 363 170**
**07868958.5 / 2 227 295**

(73) Proprietor: **Mevion Medical Systems, Inc.
Littleton, MA 01460 (US)**

(72) Inventor: **Gall, Kenneth
Harvard, MA Massachusetts 01451 (US)**

(74) Representative: **Conroy, John et al
Fish & Richardson P.C.
Highlight Business Towers
Mies-van-der-Rohe-Straße 8
80807 München (DE)**

(56) References cited:
**EP-A1- 1 419 801       CA-A1- 2 629 333
US-A1- 2006 173 294   US-A1- 2007 133 752
US-B1- 7 014 361**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

This patent application describes an inner gantry for use with a particle beam therapy system.

BACKGROUND

The design of a proton or ion radiation therapy system for a clinical environment should take account of overall size, cost, and complexity. Available space is usually limited in crowded clinical environments. Lower cost allows more systems to be deployed to reach a broader patient population. Less complexity reduces operating costs and makes the system more reliable for routine clinical use.

Other considerations may also bear on the design of such a therapy system. By configuring the system to apply the treatment to patients who are held in a stable, reproducible position (for example, lying supine on a flat table), the physician can more precisely relocate the intended target, relative to the patient's anatomy, at each treatment. Reliable reproduction of the patient's position for each treatment also can be aided using custom molds and braces fitted to the patient. With a patient in a stable, fixed position, the radiotherapy beam can be directed into the patient from a succession of angles, so that, over the course of the treatment, the radiation dose at the target is enhanced while the extraneous radiation dose is spread over non-target tissues.

Traditionally, an isocentric gantry is rotated around the supine patient to direct the radiation beam along successive paths that lie at a range of angles in a common vertical plane toward a single point (called an isocenter) within the patient. By rotating the table on which the patient lies around a vertical axis, the beam can be directed into the patient along different paths. Other techniques have been used to vary the position of the radiation source around the patient, including robotic manipulation.

CA 2 629 333 A1 discloses an accelerator mounted on a gantry to enable the accelerator to move through a range of positions around a patient on a patient support.

US 7,014,361 B1 discloses an adaptive rotator including an at least partial ring mounted for rotation on a ring mount, a gantry coupler adapted to couple the at least partial ring to a rotatable gantry, and a mechanical compensator attached to at least one of the at least partial ring, the ring mount and the gantry coupler.

US 2006/0173294 A1 discloses a radiation modulator positioner operative to cause a relative motion between a radiation modulator and a radiation source housing. US 2007/0133752 A1 discloses an imaging device including a rotator having a hollow bore for a patient to move therein and thereout.

EP 1 419 801 A1 discloses a radiation treatment apparatus including a guide and a support member.

SUMMARY

This application relates to a system as described in claim 1. Preferred embodiments are defined in the dependent claims.

In general, this patent application describes a system comprising a patient support and an outer gantry on which an accelerator is mounted. The outer gantry enables the accelerator to move through a range of positions around a patient on the patient support. The accelerator is configured to produce a proton or ion beam having an energy level sufficient to reach a target in the patient. An inner gantry comprises an aperture for directing the proton or ion beam towards the target. The system described above may include one or more of the following features, either alone or in combination.

The inner gantry may comprise an applicator for holding the aperture. The applicator may be movable along the inner gantry. The applicator may be configured to move the aperture relative to the patient. For example, the applicator may be configured to move the aperture towards, or away from, the patient.

The inner gantry may comprise a track along which the applicator is configured to move. A cover may be movable relative to the track. The cover may be for preventing objects from falling into a vault below the patient support.

A processing device may be programmed to control movement of the outer gantry and/or the inner gantry. The processing device may be configured to control movement of the outer gantry and/or the inner gantry to substantially align the proton or ion beam with the aperture. The aperture may be configured to substantially collimate the proton or ion beam. The system may comprise a patient support that is movable relative to the inner gantry and/or the outer gantry.

In general, this patent application also describes a system comprising a patient support and a gantry on which a particle beam accelerator is mounted. The particle beam accelerator is for directing a particle beam towards the patient support. The gantry is movable to positions above and below the patient support. An aperture is located between the particle beam accelerator and the patient support. The aperture is for modifying the particle beam. The system described above may include one or more of the following features, either alone or in combination.

The system may comprise an apparatus to hold the aperture. The apparatus may be movable relative to the patient support. The apparatus may comprise a robotic arm that is computer controlled to position the aperture relative to the patient support. The apparatus may comprise a stand, which is manually positionable, to hold the aperture.

The particle beam accelerator may be a synchrocyclotron. The system may comprise a second gantry that includes an applicator to hold the aperture. The second gantry may be controlled to substantially align the aperture with the particle beam.

In general, this patent application also describes a system comprising a patient support, a first gantry that is angularly movable relative to the patient support, and a particle accelerator that is mounted on the first gantry. The particle accelerator is configured to provide a particle beam directly towards the patient support. A second gantry is positioned relative to the patient support. The second gantry is substantially C-shaped. The system described above may include one or more of the following features, either alone or in combination.

The second gantry may comprise a track, an aperture, and an applicator. The applicator may be movable along the track so that the aperture is substantially aligned with the particle beam. The aperture may alter the particle beam before the particle beam reaches a patient on the patient support.

The system may comprise a computer to control the first gantry and the second gantry. The first gantry may be movable so that the particle accelerator is in a position above the patient support to a position below the patient support. The second gantry may comprise a cover to protect the particle accelerator when the particle accelerator is in the position below the patient support. The inner gantry may comprise a device to alter a size and/or shape of the particle beam. The device for altering the particle beam may be movable relative to the synchrocyclotron.

Any of the foregoing features may be combined to form implementations not specifically described herein.

The details of one or more examples are set forth in the accompanying drawings and the description below. Further features, aspects, and advantages will become apparent from the description, the drawings, and the claims.

DESCRIPTION OF THE DRAWINGS

Figure 1 is a perspective view of a therapy system.
Figure 2 is an exploded perspective view of components of a synchrocyclotron.
Figures 3, 4, and 5 are cross-sectional views of a synchrocyclotron.
Figure 6 is a perspective view of a synchrocyclotron.
Figure 7 is a cross-sectional view of a portion of a reverse bobbin and windings.
Figure 8 is a cross sectional view of a cable-in-channel composite conductor.
Figure 9 is a cross-sectional view of an ion source.
Figure 10 is a perspective view of a dee plate and a dummy dee.
Figure 11 is a perspective view of a vault.
Figure 12 is a perspective view of a treatment room with a vault.
Figure 13 shows a profile of one-half of a symmetrical profile of a pole face and a pole piece.
Figure 14 shows a patient positioned within an inner gantry in a treatment room.
Figure 15 is a perspective view showing both the outer and inner gantries positioned to apply a proton or ion beam from above the patient.
Figure 16 shows the shape of a particle beam provided by an accelerator.
Figure 17 is a perspective view showing both the outer and inner gantries positioned to apply a proton or ion beam from above below the patient.
Figure 18 shows components of the inner gantry.
Figure 19 shows a robotic arm used to perform functions of the inner gantry.

DETAILED DESCRIPTION

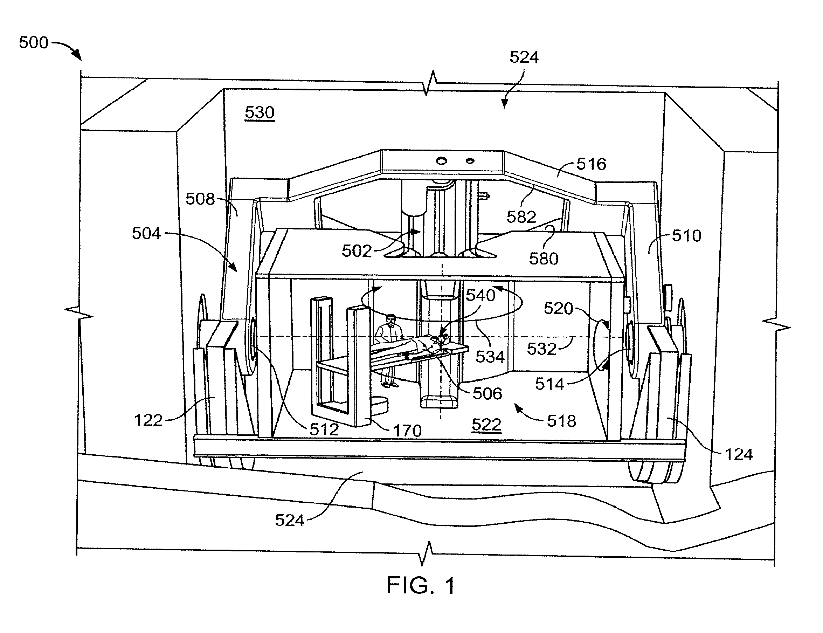

As shown in Fig. 1, a charged particle radiation therapy system 500 includes a beam-producing particle accelerator 502 having a weight and size small enough to permit it to be mounted on a rotating gantry 504 with its output directed straight (that is, essentially directly) from the accelerator housing toward a patient 506.

In some implementations, the steel gantry has two legs 508, 510 mounted for rotation on two respective bearings 512, 514 that lie on opposite sides of the patient. The accelerator is supported by a steel truss 516 that is long enough to span a treatment area 518 in which the patient lies (e.g., twice as long as a tall person, to permit the person to be rotated fully within the space with any desired target area of the patient remaining in the line of the beam) and is attached stably at both ends to the rotating legs of the gantry.

In some examples, the rotation of the gantry is limited to a range 520 of less than 360 degrees, e.g., about 180 degrees, to permit a floor 522 to extend from a wall of the vault 524 that houses the therapy system into the patient treatment area. The limited rotation range of the gantry also reduces the required thickness of some of the walls (which never directly receive the beam, e.g., wall 530), which provide radiation shielding of people outside the treatment area. A range of 180 degrees of gantry rotation is enough to cover all treatment approach angles, but providing a larger range of travel can be useful. For example the range of rotation may be between 180 and 330 degrees and still provide clearance for the therapy floor space.

The horizontal rotational axis 532 of the gantry is located nominally one meter above the floor where the patient and therapist interact with the therapy system. This floor is positioned about 3 meters above the bottom floor of the therapy system shielded vault. The accelerator can swing under the raised floor for delivery of treatment beams from below the rotational axis. The patient couch moves and rotates in a substantially horizontal plane parallel to the rotational axis of the gantry. The couch can rotate through a range 534 of about 270 degrees in the horizontal plane with this configuration. This combination of gantry and patient rotational ranges and degrees of freedom allow the therapist to select virtually any approach angle for the beam. If needed, the patient can be placed on the couch in the opposite orientation and then all possible angles can be used.

In some implementations, the accelerator uses a synchrocyclotron configuration having a very high magnetic field superconducting electromagnetic structure. Because the bend radius of a charged particle of a given kinetic energy is reduced in direct proportion to an increase in the magnetic field applied to it, the very high magnetic field superconducting magnetic structure permits the accelerator to be made smaller and lighter. The synchrocyclotron uses a magnetic field that is uniform in rotation angle and falls off in strength with increasing radius. Such a field shape can be achieved regardless of the magnitude of the magnetic field, so in theory there is no upper limit to the magnetic field strength (and therefore the resulting particle energy at a fixed radius) that can be used in a synchrocyclotron.

Certain superconducting materials begin to lose their superconducting properties in the presence of very high magnetic fields. High performance superconducting wire windings are used to allow very high magnetic fields to be achieved.

Superconducting materials typically need to be cooled to low temperatures for their superconducting properties to be realized. In some examples described here, cryo-coolers are used to bring the superconducting coil windings to temperatures near absolute zero. Using cryo-coolers can reduce complexity and cost.

The synchrocyclotron is supported on the gantry so that the beam is generated directly in line with the patient. The gantry permits rotation of the cyclotron about a horizontal rotational axis that contains a point (isocenter 540) within, or near, the patient. The split truss that is parallel to the rotational axis, supports the cyclotron on both sides.

Because the rotational range of the gantry is limited, a patient support area can be accommodated in a wide area around the isocenter. Because the floor can be extended broadly around the isocenter, a patient support table can be positioned to move relative to and to rotate about a vertical axis 542 through the isocenter so that, by a combination of gantry rotation and table motion and rotation, any angle of beam direction into any part of the patient can be achieved. The two gantry arms are separated by more than twice the height of a tall patient, allowing the couch with patient to rotate and translate in a horizontal plane above the raised floor.

Limiting the gantry rotation angle allows for a reduction in the thickness of at least one of the walls surrounding the treatment room. Thick walls, typically constructed of concrete, provide radiation protection to individuals outside the treatment room. A wall downstream of a stopping proton beam may be about twice as thick as a wall at the opposite end of the room to provide an equivalent level of protection. Limiting the range of gantry rotation enables the treatment room to be sited below earth grade on three sides, while allowing an occupied area adjacent to the thinnest wall reducing the cost of constructing the treatment room.

In the example implementation shown in Fig. 1, the superconducting synchrocyclotron 502 operates with a peak magnetic field in a pole gap of the synchrocyclotron of 8.8 Tesla. The synchrocyclotron produces a beam of protons having an energy of 250 MeV. In other implementations the field strength could be in the range of 6 to 20 Tesla and the proton energy could be in the range of 150 to 300 MeV

The radiation therapy system described in this example is used for proton radiation therapy, but the same principles and details can be applied in analogous systems for use in heavy ion (ion) treatment systems.

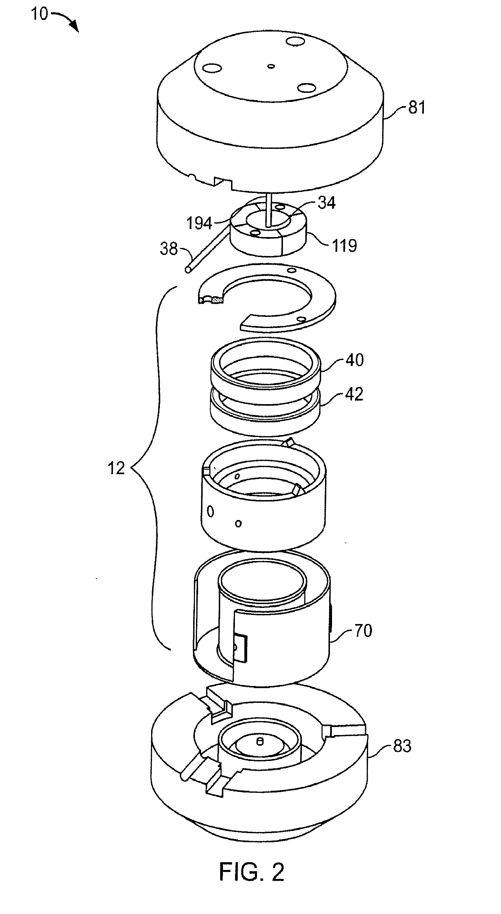

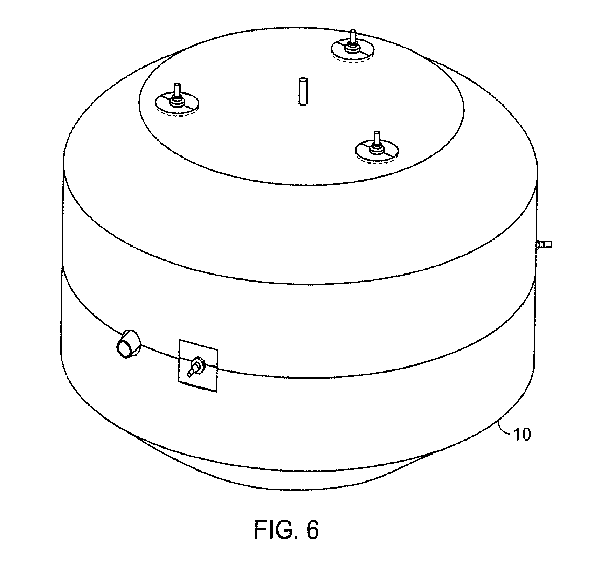

As shown in Figs. 2, 3, 4, 5, and 6, an example synchrocyclotron 10 (502 in Fig. 1) includes a magnet system 12 that contains an ion source 90, a radiofrequency drive system 91, and a beam extraction system 38. The magnetic field established by the magnet system has a shape appropriate to maintain focus of a contained proton beam using a combination of a split pair of annular superconducting coils 40, 42 and a pair of shaped ferromagnetic (e.g., low carbon steel) pole faces 44, 46.

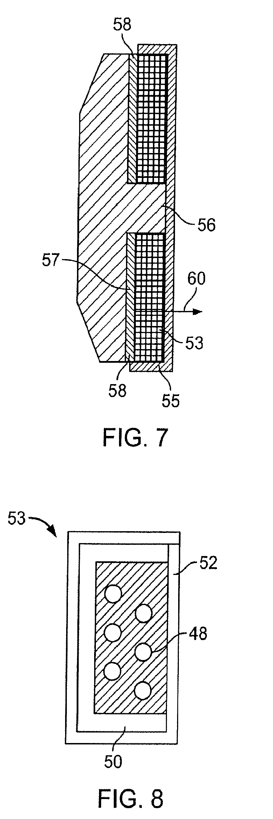

The two superconducting magnet coils are centered on a common axis 47 and are spaced apart along the axis. As shown in Figs. 7 and 8, the coils are formed by of Nb3Sn-based superconducting 0.6 mm diameter strands 48 (that initially comprise a niobium-tin core surrounded by a copper sheath) deployed in a Rutherford cable-in-channel conductor geometry. After six individual strands are laid in a copper channel 50, they are heated to cause a reaction that forms the final (brittle) material of the winding. After the material has been reacted, the wires are soldered into the copper channel (outer dimensions 3.02 x 1.96 mm and inner dimensions 2.05 x 1.27 mm) and covered with insulation 52 (in this example, a woven fiberglass material). The copper channel containing the wires 53 is then wound in a coil having a rectangular cross-section of 6.0 cm x 15.25 cm, having 30 layers and 47 turns per layer. The wound coil is then vacuum impregnated with an epoxy compound 54. The finished coils are mounted on an annular stainless steel reverse bobbin 56. A heater blanket 55 is held against the inner face of the bobbin and the windings to protect the assembly in the event of a magnet quench. In an alternate version, the superconducting coil may be formed of 0.8 mm diameter Nb3Sn based strands. These strands can be deployed in a 4 strand cable, heat treated to form the superconducting matrix and soldered into a copper channel of outer dimension 3.19 by 2.57 mm. The integrated cable in channel conductor can be insulated with overlapped woven fiberglass tape and then wound into coils of 49 turns and 26 layers deep with a rectangular cross section of 79.79 mm by 180.5 mm and inner radius of 374.65 mm. The wound coil is then vacuum impregnated with an epoxy compound. The entire coil can then be covered with copper sheets to provide thermal conductivity and mechanical stability and then contained in an additional layer of epoxy. The precompression of the coil can be provided by heating the stainless steel reverse bobbin and fitting the coils within the reverse bobbin. The reverse bobbin inner diameter is chosen so that when the entire mass is cooled to 4 K, the reverse bobbin stays in contact with the coil and provides some compression. Heating the stainless steel reverse bobbin to approximately 50 degrees C and fitting coils at room temperature (20 degrees C) can achieve this.

The geometry of the coil is maintained by mounting the coils in a "reverse" rectangular bobbin 56 and incorporating a pre-compression stainless steel bladder 58 between each coil and an inner face 57 of the bobbin to exert a restorative force 60 that works against the distorting force produced when the coils are energized. The bladder is pre-compressed after the coils and the heater blanket are assembled on the bobbin, by injecting epoxy into the bladder and allowing it to harden. The precompression force of the bladder is set to minimize the strain in the brittle Nb3Sn superconducting matrix through all phases of cool-down and magnet energizing.

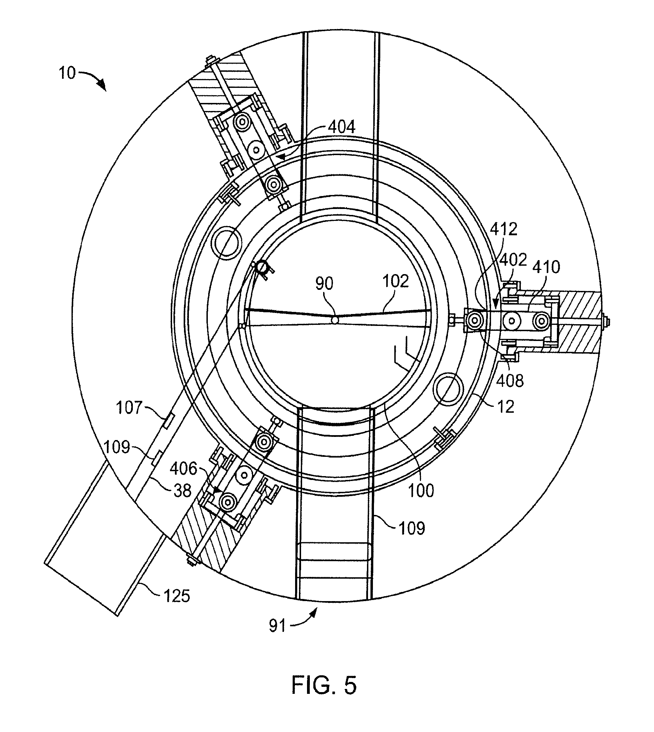

As shown in Fig. 5, the coil position is maintained relative to the magnet yoke and cryostat using a set of warm-to-cold support straps 402, 404, 406. Supporting the cold mass with thin straps reduces the heat leakage imparted to the cold mass by the rigid support system. The straps are arranged to withstand the varying gravitational force on the coil as the magnet rotates on board the gantry. They withstand the combined effects of gravity and the large de-centering force realized by the coil when it is perturbed from a perfectly symmetric position relative to the magnet yoke. Additionally the links act to reduce dynamic forces imparted on the coil as the gantry accelerates and decelerates when its position is changed. Each warm-to-cold support includes 3 S2 fiberglass links. Two links 410, 412 are supported across pins between the warm yoke and an intermediate temperature (50 - 70 K), and one link 408 is supported across the intermediate temperature pin and a pin attached to the cold mass. Each link is 10.2 cm long (pin center to pin center) and is 20 mm wide. The link thickness is 1.59 mm. Each pin is made of stainless steel and is 47.7 mm in diameter.

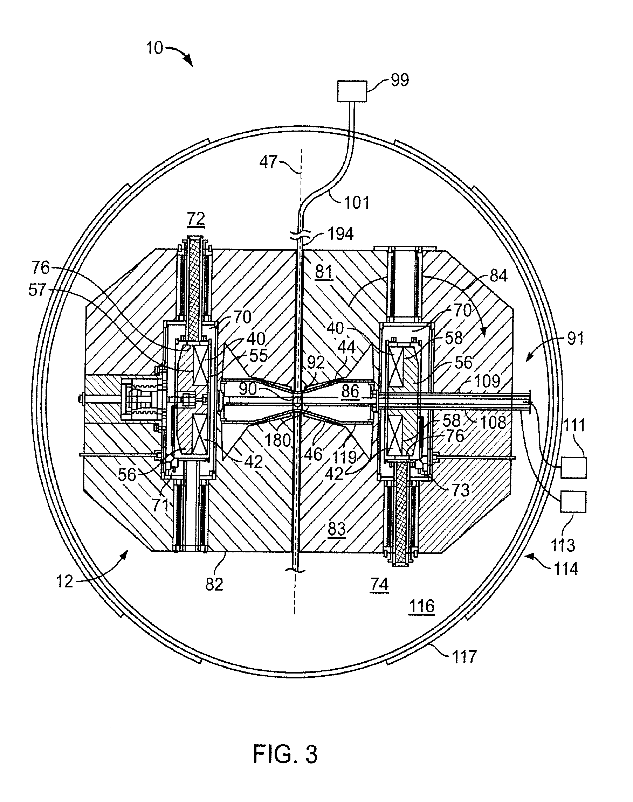

Referring to Fig. 3, the field strength profile as a function of radius is determined largely by choice of coil geometry; the pole faces 44, 46 of the permeable yoke material can be contoured to fine tune the shape of the magnetic field to ensure that the particle beam remains focused during acceleration.

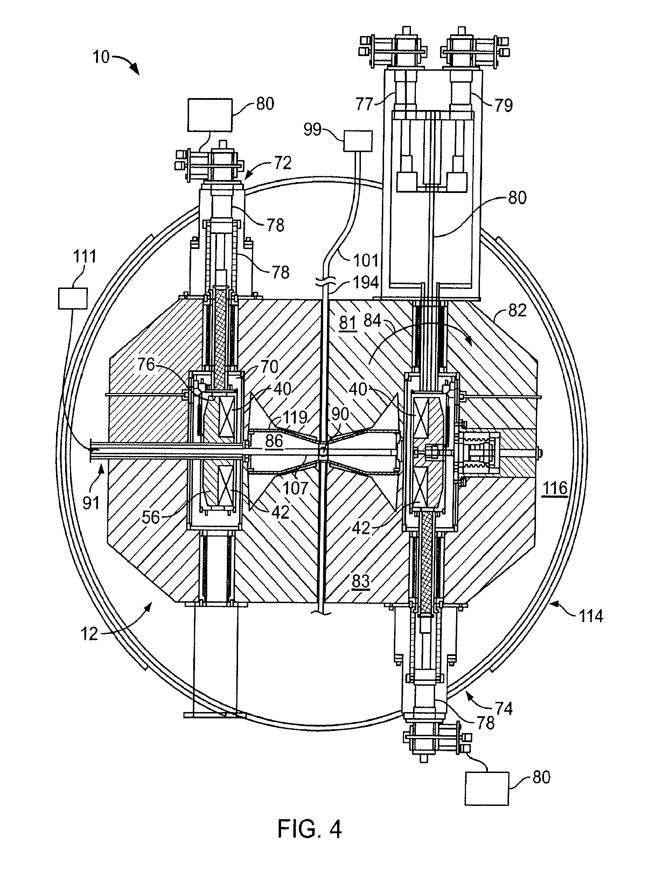

The superconducting coils are maintained at temperatures near absolute zero (e.g., about 4 degrees Kelvin) by enclosing the coil assembly (the coils and the bobbin) inside an evacuated annular aluminum or stainless steel cryostatic chamber 70 that provides a free space around the coil structure, except at a limited set of support points 71, 73. In an alternate version (Fig. 4) the outer wall of the cryostat may be made of low carbon steel to provide an additional return flux path for the magnetic field. The temperature near absolute zero is achieved and maintained using two Gifford-McMahon cryo-coolers 72, 74 that are arranged at different positions on the coil assembly. Each cryo-cooler has a cold end 76 in contact with the coil assembly. The cryo-cooler heads 78 are supplied with compressed Helium from a compressor 80. Two other Gifford-McMahon cryo-coolers 77, 79 are arranged to cool high temperature (e.g., 60 - 80 degrees Kelvin) leads 81 that supply current to the superconducting windings.

The coil assembly and cryostatic chambers are mounted within and fully enclosed by two halves 81, 83 of a pillbox-shaped magnet yoke 82. In this example, the inner diameter of the coil assembly is about 140 cm. The iron yoke 82 provides a path for the return magnetic field flux 84 and magnetically shields the volume 86 between the pole faces 44, 46 to prevent external magnetic influences from perturbing the shape of the magnetic field within that volume. The yoke also serves to decrease the stray magnetic field in the vicinity of the accelerator.

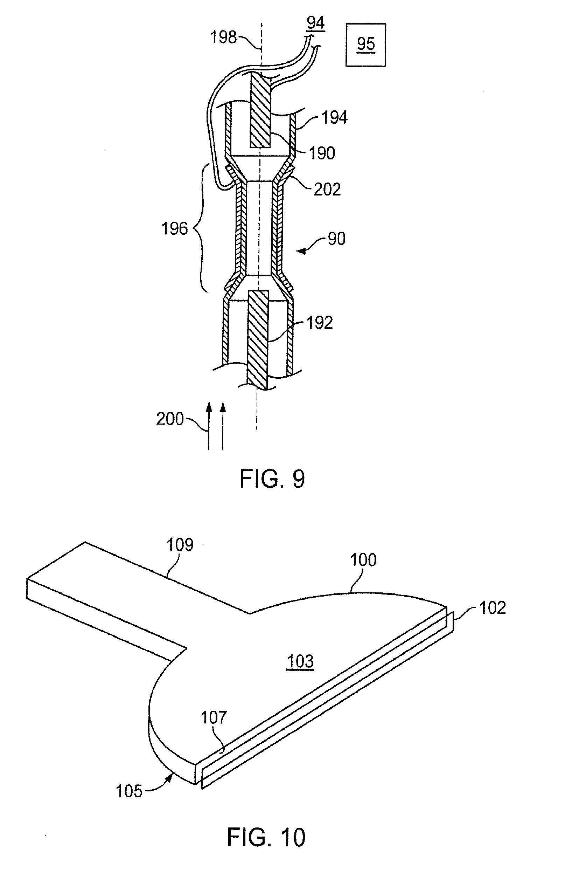

As shown in Figs. 3 and 9, the synchrocyclotron includes an ion source 90 of a Penning ion gauge geometry located near the geometric center 92 of the magnet structure 82. The ion source may be as described below.

Ion source 90 is fed from a supply 99 of hydrogen through a gas line 101 and tube 194 that delivers gaseous hydrogen. Electric cables 94 carry an electric current from a current source 95 to stimulate electron discharge from cathodes 192, 190 that are aligned with the magnetic field, 200.

In this example, the discharged electrons ionize the gas exiting through a small hole from tube 194 to create a supply of positive ions (protons) for acceleration by one semicircular (dee-shaped) radio-frequency plate 100 that spans half of the space enclosed by the magnet structure and one dummy dee plate 102. In the case of an interrupted ion source, all (or a substantial part) of the tube containing plasma is removed at the acceleration region, thereby allowing ions to be more rapidly accelerated in a relatively high magnetic field.

As shown in Fig. 10, the dee plate 100 is a hollow metal structure that has two semicircular surfaces 103, 105 that enclose a space 107 in which the protons are accelerated during half of their rotation around the space enclosed by the magnet structure. A duct 109 opening into the space 107 extends through the yoke to an external location from which a vacuum pump 111 can be attached to evacuate the space 107 and the rest of the space within a vacuum chamber 119 in which the acceleration takes place. The dummy dee 102 comprises a rectangular metal ring that is spaced near to the exposed rim of the dee plate. The dummy dee is grounded to the vacuum chamber and magnet yoke. The dee plate 100 is driven by a radio-frequency signal that is applied at the end of a radio-frequency transmission line to impart an electric field in the space 107. The radio frequency electric field is made to vary in time as the accelerated particle beam increases in distance from the geometric center. Examples of radio frequency waveform generators that are useful for this purpose are described in U.S. Patent Application No. 11/187,633, titled "A Programmable Radio Frequency Waveform Generator for a Synchrocyclotron," filed July 21, 2005, and in U.S. Provisional Application No. 60/590,089, same title, filed on July 21, 2004.

For the beam emerging from the centrally located ion source to clear the ion source structure as it begins to spiral outward, a large voltage difference is required across the radio frequency plates. 20,000 Volts is applied across the radio frequency plates. In some versions from 8,000 to 20,000 Volts may be applied across the radio frequency plates. To reduce the power required to drive this large voltage, the magnet structure is arranged to reduce the capacitance between the radio frequency plates and ground. This is done by forming holes with sufficient clearance from the radio frequency structures through the outer yoke and the cryostat housing and making sufficient space between the magnet pole faces.

The high voltage alternating potential that drives the dee plate has a frequency that is swept downward during the accelerating cycle to account for the increasing relativistic mass of the protons and the decreasing magnetic field. The dummy dee does not require a hollow semi-cylindrical structure as it is at ground potential along with the vacuum chamber walls. Other plate arrangements could be used such as more than one pair of accelerating electrodes driven with different electrical phases or multiples of the fundamental frequency. The RF structure can be tuned to keep the Q high during the required frequency sweep by using, for example, a rotating capacitor having intermeshing rotating and stationary blades. During each meshing of the blades, the capacitance increases, thus lowering the resonant frequency of the RF structure. The blades can be shaped to create a precise frequency sweep required. A drive motor for the rotating condenser can be phase locked to the RF generator for precise control. One bunch of particles is accelerated during each meshing of the blades of the rotating condenser.

The vacuum chamber 119 in which the acceleration occurs is a generally cylindrical container that is thinner in the center and thicker at the rim. The vacuum chamber encloses the RF plates and the ion source and is evacuated by the vacuum pump 111. Maintaining a high vacuum insures that accelerating ions are not lost to collisions with gas molecules and enables the RF voltage to be kept at a higher level without arcing to ground.

Protons traverse a generally spiral path beginning at the ion source. In half of each loop of the spiral path, the protons gain energy as they pass through the RF electric field in space 107. As the ions gain energy, the radius of the central orbit of each successive loop of their spiral path is larger than the prior loop until the loop radius reaches the maximum radius of the pole face. At that location a magnetic and electric field perturbation directs ions into an area where the magnetic field rapidly decreases, and the ions depart the area of the high magnetic field and are directed through an evacuated tube 38 to exit the yoke of the cyclotron. The ions exiting the cyclotron will tend to disperse as they enter the area of markedly decreased magnetic field that exists in the room around the cyclotron. Beam shaping elements 107, 109 in the extraction channel 38 redirect the ions so that they stay in a straight beam of limited spatial extent.

The magnetic field within the pole gap needs to have certain properties to maintain the beam within the evacuated chamber as it accelerates. The magnetic field index n, which is shown below,

$$n = -(r/B)dB/dr,$$

should be kept positive to maintain this "weak" focusing. Here r is the radius of the beam and B is the magnetic field. Additionally the field index needs to be maintained below 0.2, because at this value the periodicity of radial oscillations and vertical oscillations of the beam coincide in a $v_r = 2 v_z$ resonance. The betatron frequencies are defined by $v_r = (1-n)^{1/2}$ and $v_z = n^{1/2}$. The ferromagnetic pole face is designed to shape the magnetic field generated by the coils so that the field index n is maintained positive and less than 0.2 in the smallest diameter consistent with a 250 MeV beam in the given magnetic field.

As the beam exits the extraction channel it is passed through a beam formation system 125 (Fig. 5) that can be programmably controlled to create a desired combination of scattering angle and range modulation for the beam. Examples of beam forming systems useful for that purpose are described in U.S. Patent Application No. 10/949,734, titled "A Programmable Particle Scatterer for Radiation Therapy Beam Formation", filed September 24, 2004, and U.S. Provisional Application No.60/590,088, filed July 21, 2005. Beam formation system 125 may be used in conjunction with an inner gantry 601, which is described below, to direct a beam to the patient.

During operation, the plates absorb energy from the applied radio frequency field as a result of conductive resistance along the surfaces of the plates. This energy appears as heat and is removed from the plates using water cooling lines 108 that release the heat in a heat exchanger 113 (Fig. 3).

Stray magnetic fields exiting from the cyclotron are limited by both the pillbox magnet yoke (which also serves as a shield) and a separate magnetic shield 114. The separate magnetic shield includes of a layer 117 of ferromagnetic material (e.g., steel or iron) that encloses the pillbox yoke, separated by a space 116. This configuration that includes a sandwich of a yoke, a space, and a shield achieves adequate shielding for a given leakage magnetic field at lower weight.

As mentioned, the gantry allows the synchrocyclotron to be rotated about the horizontal rotational axis 532. The truss structure 516 has two generally parallel spans 580, 582. The synchrocyclotron is cradled between the spans about midway between the legs. The gantry is balanced for rotation about the bearings using counterweights 122, 124 mounted on ends of the legs opposite the truss.

The gantry is driven to rotate by an electric motor mounted to one of the gantry legs and connected to the bearing housings by drive gears and belts or chains. The rotational position of the gantry is derived from signals provided by shaft angle encoders incorporated into the gantry drive motors and the drive gears.

At the location at which the ion beam exits the cyclotron, the beam formation system 125 acts on the ion beam to give it properties suitable for patient treatment. For example, the beam may be spread and its depth of penetration varied to provide uniform radiation across a given target volume. The beam formation system can include passive scattering elements as well as active scanning elements.

All of the active systems of the synchrocyclotron (the current driven superconducting coils, the RF-driven plates, the vacuum pumps for the vacuum acceleration chamber and for the superconducting coil cooling chamber, the current driven ion source, the hydrogen gas source, and the RF plate coolers, for example), are controlled by appropriate synchrocyclotron control electronics (not shown), which may include, e.g., a computer programmed with appropriate programs to effect control.

The control of the gantry, the patient support, the active beam shaping elements, and the synchrocyclotron to perform a therapy session is achieved by appropriate therapy control electronics (not shown).

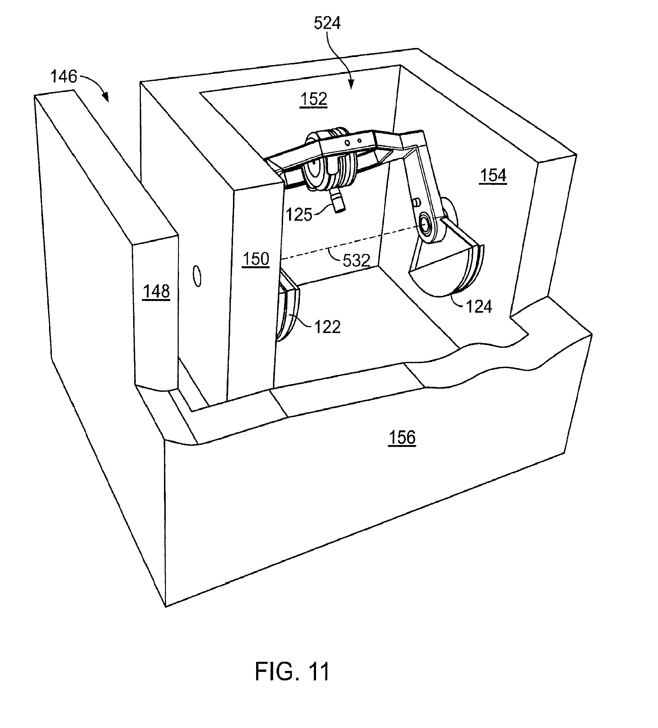

As shown in Figs. 1, 11, and 12, the gantry bearings are supported by the walls of a cyclotron vault 524. The gantry enables the cyclotron to be swung through a range 520 of 180 degrees (or more) including positions above, to the side of, and below the patient. The vault is tall enough to clear the gantry at the top and bottom extremes of its motion. A maze 146 sided by walls 148, 150 provides an entry and exit route for therapists and patients. Because at least one wall 152 is never in line with the proton beam directly from the cyclotron, it can be made relatively thin and still perform its shielding function. The other three side walls 154, 156, 150/148 of the room, which may need to be more heavily shielded, can be buried within an earthen hill (not shown). The required thickness of walls 154, 156, and 158 can be reduced, because the earth can itself provide some of the needed shielding.

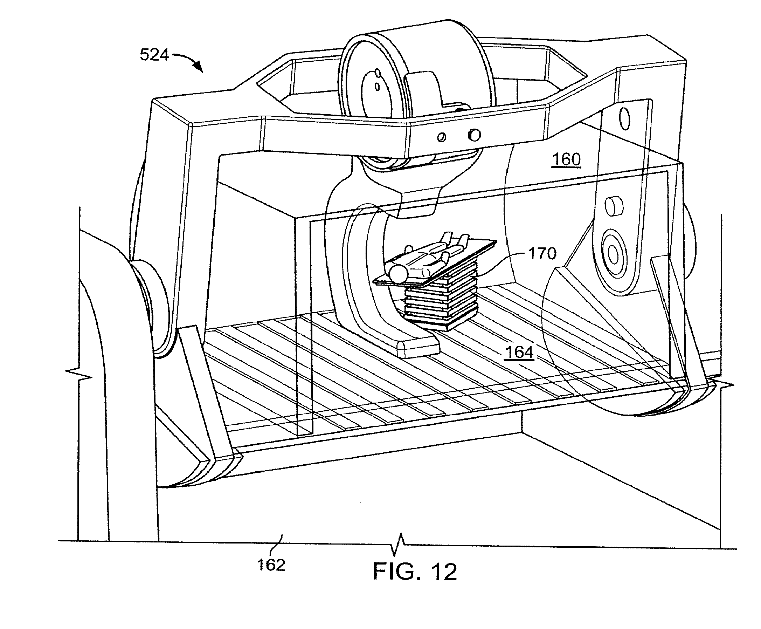

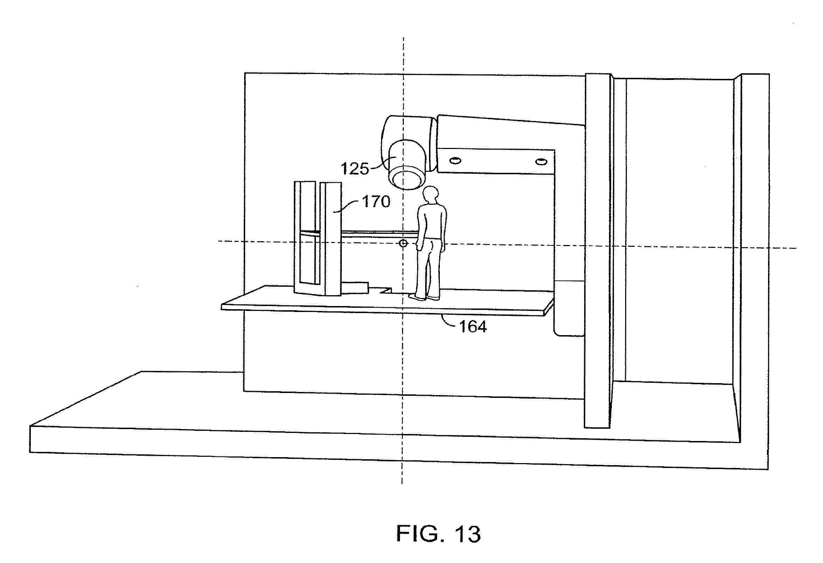

Referring to Figs. 12 and 13, for safety and aesthetic reasons, a therapy room 160 may be constructed within the vault. The therapy room is cantilevered from walls 154, 156, 150 and the base 162 of the containing room into the space between the gantry legs in a manner that clears the swinging gantry and also maximizes the extent of the floor space 164 of the therapy room. Periodic servicing of the accelerator can be accomplished in the space below the raised floor. When the accelerator is rotated to the down position on the gantry, full access to the accelerator is possible in a space separate from the treatment area. Power supplies, cooling equipment, vacuum pumps and other support equipment can be located under the raised floor in this separate space.

Within the treatment room, the patient support 170 can be mounted in a variety of ways that permit the support to be raised and lowered and the patient to be rotated and moved to a variety of positions and orientations.

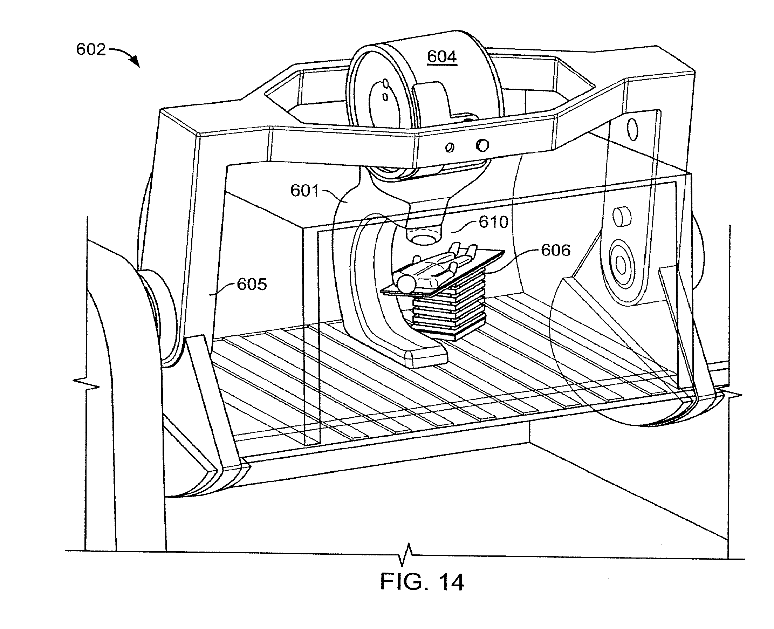

In system 602 of Fig. 14, a beam-producing particle accelerator, in this case synchrocyclotron 604, is mounted on rotating gantry 605. Rotating gantry 605 is of the type described herein, and can angularly rotate around patient support 606. This feature enables synchrocyclotron 604 to provide a particle beam directly to the patient from various angles. For example, as in Fig. 14, if synchrocyclotron 604 is above patient support 606, the particle beam may be directed downwards toward the patient. Alternatively, if synchrocyclotron 604 is below patient support 606, the particle beam may be directed upwards toward the patient. The particle beam is applied directly to the patient in the sense that an intermediary beam routing mechanism is not required. A routing mechanism, in this context, is different from a shaping or sizing mechanism in that a shaping or sizing mechanism does not re-route the beam, but rather sizes and/or shapes the beam while maintaining the same general trajectory of the beam.

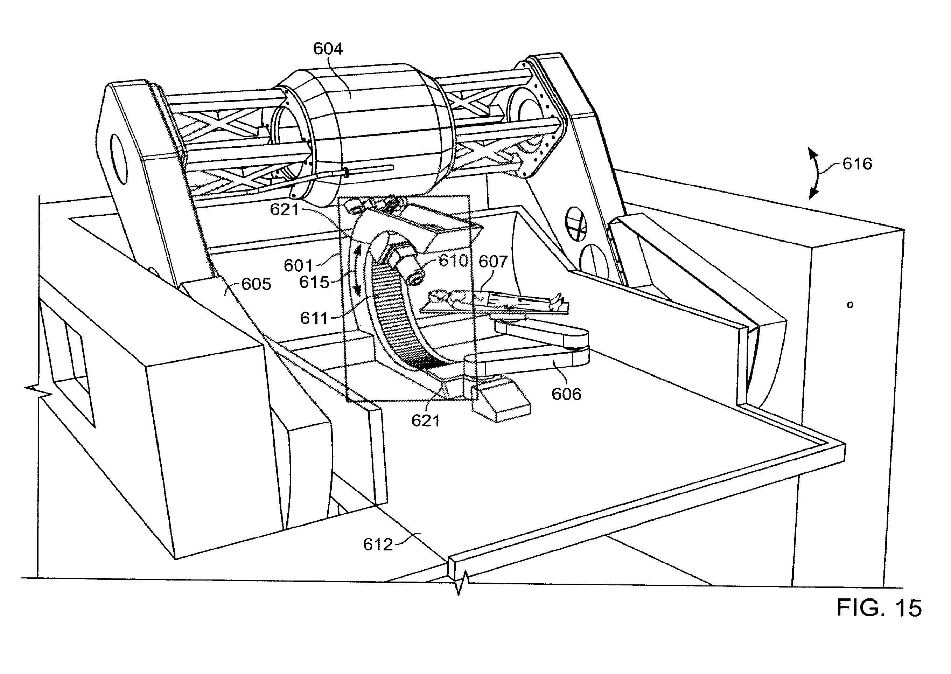

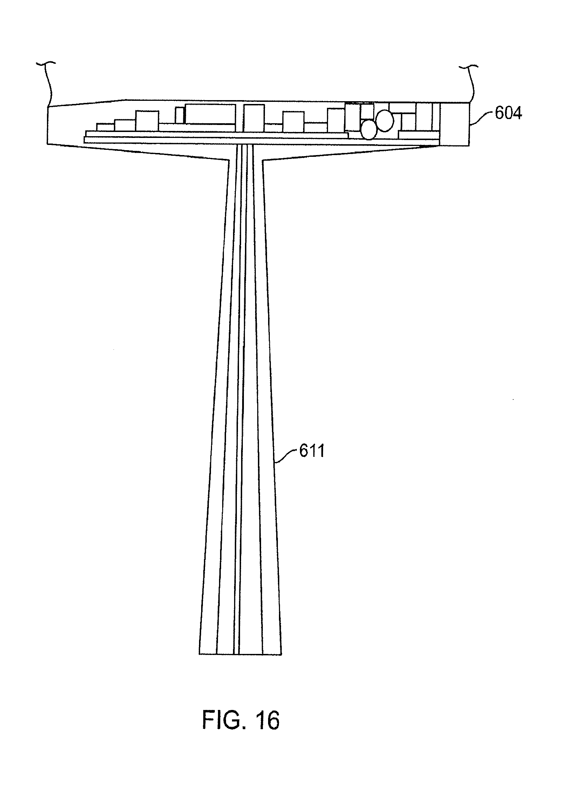

Referring also to Fig. 15, an inner gantry 601 may be included system 602. In this example, inner gantry 601 is roughly C-shaped, as shown. Inner gantry 601 includes an applicator 610. Applicator 610 is mounted in a manner that permits applicator 610 to move along the surface 611 of inner gantry 601 relative to patient support 606 (which is a different type of support than that depicted in Fig. 12). This enables the applicator to be positioned anywhere within, e.g., a half-circle around the patient, e.g., anywhere above, alongside, or below the patient 607. Applicator 610 may alter the particle beam provided by synchrocyclotron 604. More specifically, as shown in Fig. 16, the particle beam 611 provided by the beam shaping system of synchrocyclotron 604 may diverge the further the particle beam gets from the output of synchrocyclotron 604. Applicator 610 may receive the particle beam from the output of synchrocyclotron 604 and alter characteristics of the particle beam. For example, applicator 610 may include an aperture and/or other beam-focusing mechanisms to substantially collimate the particle beam. As a result, the particle beam can be more precisely applied to a target in the patient. For example, the particle beam can be sized and/or shaped to treat tumors of specific sizes and/or shapes. In this regard, applicator 610 is not limited to collimating the particle beam. For example, applicator 610 may reduce the size of the particle beam while also collimating the beam. The applicator may be a multi-leaf collimator for sizing and/or shaping the particle beam. Applicator 610 may also simply allow the particle beam to pass without alteration. Applicator 610 may be computer controlled to affect the size and/or shape of the beam, as desired.

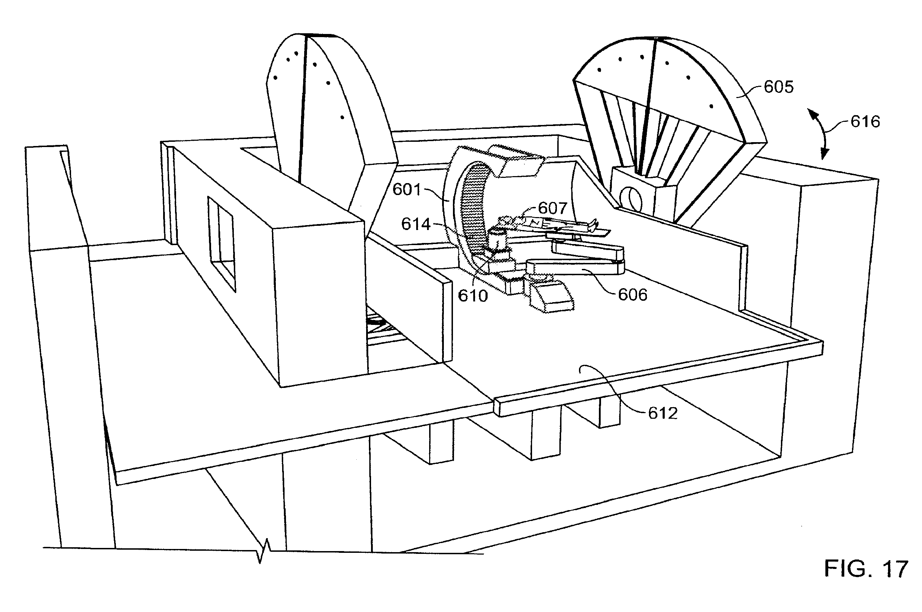

Applicator 610 and synchrocyclotron 604 may move relative to patient support 606 (and thus the patient) and relative to one another. For example, movement of applicator 610 may substantially coincide with rotation of gantry 605, or one may follow the other, so that the output of synchrocyclotron 604 aligns to the input of applicator 610. Figs. 15 and 17 illustrate movement of gantry 605 and movement of applicator 610 along inner gantry 601. More specifically, Fig. 17 shows a case where gantry 605 is rotated such that synchrocyclotron 604 is in a vault below patient support 606. In Fig. 17, synchrocyclotron 604 is below the floor 612 of the treatment room, which floor may be made of concrete. Therefore, synchrocyclotron 604 is not visible in Fig. 17. In this case, applicator 610 is moved along inner gantry 601 so that applicator 610 aligns to the output of synchrocyclotron 604. Because synchrocyclotron 604 is not shown in Fig. 17, this alignment is not visible. Nevertheless, a particle beam output from synchrocyclotron 604 passes through cover 614 of inner gantry 601 and a corresponding hole in the floor (not shown) and is thereafter is received by applicator 610. Applicator 610 performs any alteration on the particle beam, and passes the particle beam to patient 607.

Gantry 605 (and thus synchrocyclotron 604) is rotatable relative to the patient in the directions of arrow 615. Applicator 610 is movable along inner gantry 601 in the directions of arrow 616. Fig. 15 shows the locations of synchrocyclotron 604 and applicator 610 after the movements depicted by arrows 615 and 616, respectively. In Fig. 15, both synchrocyclotron 604 and applicator 610 are above patient support 606 (and thus above patient 607). In this configuration, synchrocyclotron 604 directs its particle beam downward, toward the patient. Applicator 610 receives the particle beam, alters (e.g., collimates) the particle beam, and passes the resulting particle beam to the patient.

Patient support 606 is movable relative to inner gantry 601, thereby enabling the patient to be moved such that a top part 621 of inner gantry 601 is above the patient, and such that a bottom part 622 of inner gantry 601 is below the patient. Movement of patient support 606, along with movement of gantry 605 and applicator 610, enables relatively precise targeting of tumors and/or other treatment areas on the patient.

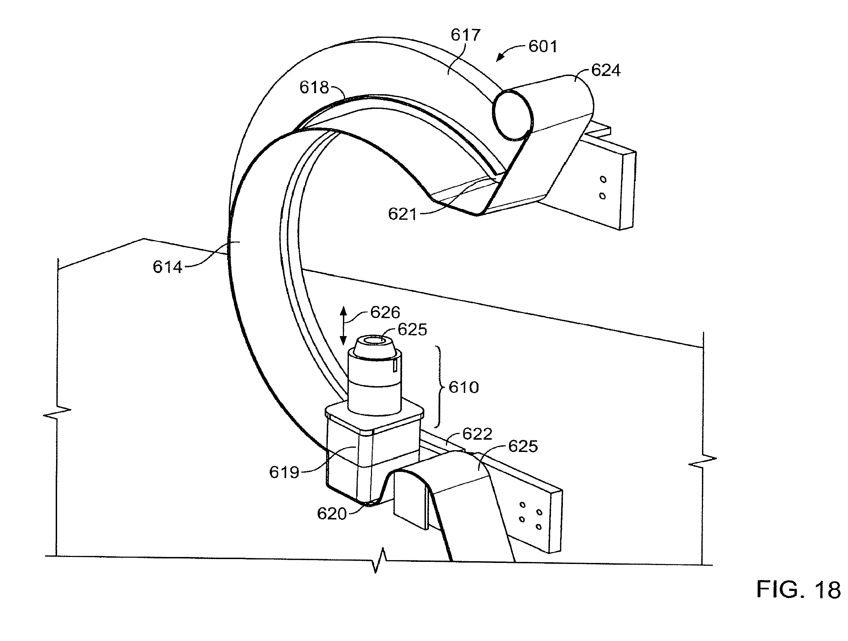

Fig. 18 shows an example construction of inner gantry 601. In this example, inner gantry includes a structural weldment 617, a precision linear bearing rail 618 (e.g., a THK rail), cover 614, and applicator 610 that includes an extension drive 619, and a theta drive 620.

Inner gantry 601 may include features in addition to those show, substitutions for the features that are shown, or both.

Structural weldment 617 may be constructed of any rigid material, such as metal, plastic, or the like, which is capable of supporting the weight of applicator 610. In this example, structural weldment 617 is substantially C-shaped (thereby defining the shape of inner gantry 601). It is noted, however, that structural weldment 617 may have other shapes. For example, it may be elongated or compressed. Basically, structural weldment may have any shape that enables relatively unobstructed, continuous travel of applicator 610 between positions that are above and below the patient.

Structural weldment 617 includes one or more bearing rails 618. The number of rails that may be used depends upon the connection required to applicator 610. Applicator 610 moves along bearing rail 618 between a top part 621 of structural weldment 617 and a bottom part 622 of structural weldment 617. The movement may be continuous or in discrete increments and may be stopped at any point along bearing rail 618 in order to obtain a desired position of applicator 610 relative to the position of the patient.

Cover 614 covers what would otherwise be an open hole to the area below floor 612 (see Fig. 17). The hole and cover allow a particle beam to pass from the synchrocyclotron to the applicator. Cover 614, however, prevents objects and/or other material from falling through that hole and possibly damaging sensitive equipment, such as the synchrocyclotron. Cover 614 may assist in, or control, movement of applicator 610 along bearing rail 618. That is, cover 614 may roll along a path between the top part 621 and the bottom part 622 of structural weldment 617. Cover 614 may roll-up at its ends 624 and/or 625, as shown in Fig. 18.

Applicator 610 includes extension drive 619 and theta drive 620. Extension drive 619 moves aperture 625 towards, and away from, the patent, e.g., along arrow 626. By virtue of this movement, extension drive may modify the projection of the aperture 625 on the patient. For example, the size of the aperture may be increased or decreased. The shape of the aperture may be altered as well, e.g., between a circular shape, an oval shape, a polygonal shape, etc. Theta drive 620 moves applicator 610 along rail 618 between top part 621 and bottom part 622 of structural weldment 617. Cover 614 may travel along with applicator 610.

All or part of extension drive 619 and theta drive 620 may be computer-controlled. For example, extension drive 619 and/or theta drive 620 may be controlled by the same hardware and/or software that is used to control gantry 605.

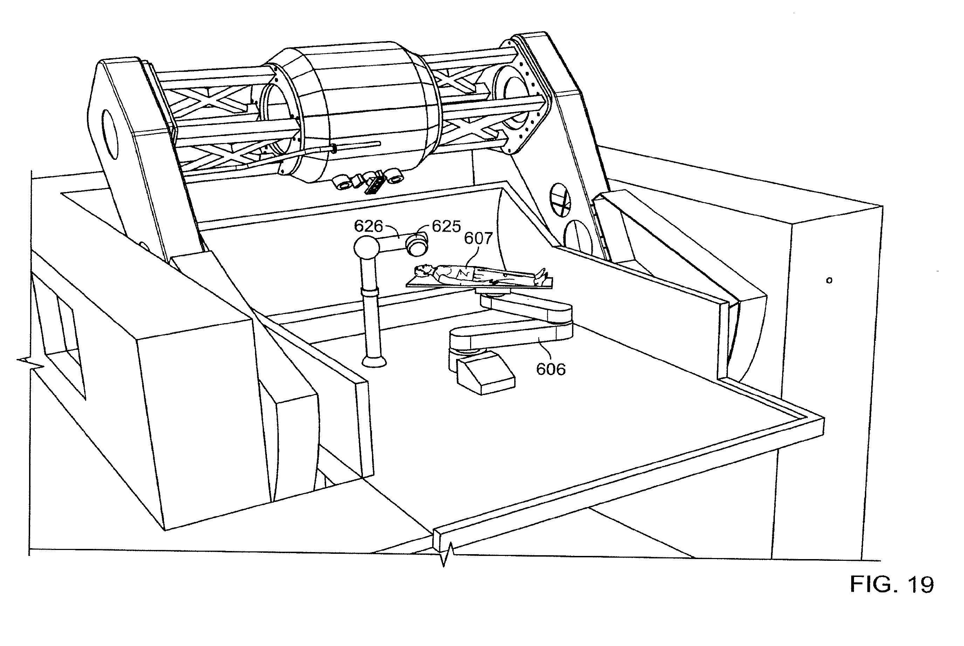

System 602 is not limited to use with inner gantry 601. Any other mechanism may be used to provide an aperture to size and/or shape (e.g., collimate) a particle beam provided by synchrocyclotron 604. For example, referring to Fig. 19, a robotic arm 626 may be used to position an aperture 625 between synchrocyclotron 604 and the patient. The robotic arm may move the aperture in three dimensions (e.g., XYZ Cartesian coordinates) relative to the patent. The robotic arm may be controlled by the same hardware and/or software that is used to control gantry 605. Additionally, the aperture itself may be controlled so that its size and/or shape is modified. As described above, the size of the aperture may be increased or decreased. The shape of the aperture may be altered as well, e.g., between a circular shape, an oval shape, a polygonal shape, etc.

An aperture, such as those described above, may be positioned and/or controlled manually. For example, a stand (not shown) may be used to hold the aperture. The aperture may be sized and/or shaped and placed on the stand. Both the stand and the aperture may be positioned relative to the patent and in line with the particle beam provided by the synchrocyclotron. Any mechanism to hold the aperture may be used. In some implementations, the aperture and/or device used to hold the aperture may be mounted to the synchrocyclotron itself.

The inner gantry is advantageous in that it reduces the precision with which the outer gantry must rotate. For example, the inner gantry allows sub-millimeter beam positioning. Because of the additional precision added by the inner gantry, the outer gantry need not provide sub-millimeter precision, but rather its precision may be at, or greater than, a millimeter. The outer gantry also need not be as large as would otherwise be required in order to obtain high levels of precision.

Additional information concerning the design of the particle accelerator described herein can be found in U.S. Provisional Application No. 60/760,788, entitled "High-Field Superconducting Synchrocyclotron" and filed January 20, 2006; U.S. Patent Application No. 11/463,402, entitled "Magnet Structure For Particle Acceleration" and filed August 9, 2006; and U.S. Provisional Application No. 60/850,565, entitled "Cryogenic Vacuum Break Pneumatic Thermal Coupler" and filed October 10, 2006.

**Claims**

1. A system comprising:

   a patient support (606) for holding a patient, the patient support being above a floor (612);
   a particle accelerator (604);
   an outer gantry (605) that is movable around the patient support (606), the particle accelerator (604) being mounted on the outer gantry (605) and
   being configured to output a proton or ion beam, the outer gantry (605) being movable so that the particle accelerator is below the floor (612); and
   an inner gantry (601) comprising a structural weldment (617) comprising a bearing rail (618), the inner gantry (601) comprising an applicator (610) mounted to the rail (618), the applicator comprising an aperture (625) for directing the proton or ion beam towards a target in the patient,
   the inner gantry (601) being configured to move the applicator (610) along the rail (618) through a range of positions around the patient so as to position the proton or ion beam at sub-millimeter precision, and
   the inner gantry (601) comprising a cover (614) configured to roll along a path between a top part (621) of the structural weldment (617) and a bottom part (622) of the structural weldment (617), the cover (614) for covering a hole in the floor above the particle accelerator (604).

2. The system of claim 1, wherein the applicator (610) is configured to move the aperture (625) towards, or away from, the patient.

3. The system of claim 1, wherein the inner gantry (601) is configured to move the applicator (610) along the rail (618) in a continuous manner.

4. The system of claim 1, wherein the inner gantry (601) is configured to move the applicator (610) along the rail (618) in discrete movements.

5. The system of claim 1, further comprising:

   a processing device programmed to control movement of the outer gantry (605) and the inner gantry (601).

6. The system of claim 5, wherein the processing device is configured to control movement of the outer gantry (605) and the inner gantry (601) to substantially align the proton or ion beam with the aperture (625).

7. The system of claim 1, wherein the aperture (625) is configured to substantially collimate the proton or ion beam.

8. The system of claim 1, wherein the patient support (606) is movable relative to the inner gantry (601) and the outer gantry (605).

9. The system of claim 1,
   wherein the applicator (610) comprises:

   an extension drive (619) to move the aperture (625) towards, or away from, a patient on the patient support (606); and
   a theta drive (620) to move the applicator (610) along different positions of the rail (618).

**10.** The system of claim 9, wherein the aperture (625) is configured to change a cross-sectional shape of the particle beam.

**11.** The system of claim 1, wherein the applicator (610) comprises a multi-leaf collimator configured to size and/or to shape the particle beam or to allow the particle beam to pass without alteration.

**Patentansprüche**

**1.** System umfassend:

einen Patienten-Träger (606) zum Tragen eines Patienten, wobei der Patienten-Träger oberhalb eines Bodens (612) angeordnet ist;
einen Teilchenbeschleuniger (604);
ein äußeres Gerüst (605) das um den Patienten-Träger (606) bewegbar ist,
wobei der Teilchenbeschleuniger (604) auf dem äußeren Gerüst (605) befestigt ist und ausgelegt ist einen Protonen- oder Ionenstrahl auszugeben, wobei das äußere Gerüst (605) bewegbar ist, so dass der Teilchenbeschleuniger sich unterhalb des Bodens (612) befindet; und
ein inneres Gerüst (601) umfassend ein konstruktives Schweißteil (617) umfassend eine Tragführung (618),
wobei das innere Gerüst (601) einen Applikator (610) umfasst, der an der Führung (618) befestigt ist, wobei der Applikator eine Apertur (625) umfasst, um den Proton- oder Ionenstrahl auf ein Ziel in dem Patienten zu richten,
wobei das innere Gerüst (601) ausgelegt ist, um den Applikator (610) entlang der Führung (618) über eine Bandbreite an Positionen um den Patienten zu bewegen, um den Protonen- oder Ionenstrahl mit eine Präzision im Sub-Millimeterbereich zu positionieren, und
wobei das innere Gerüst (601) eine Abdeckung (614) umfasst, die ausgelegt ist sich entlang eines Pfads zwischen einem oberen Teil (621) des konstruktiven Schweißteils (617) und einem unteren Teil (622) des konstruktiven Schweißteils (617) aufzurollen, wobei die Abdeckung (614) dazu dient, eine Öffnung in dem Boden über dem Teilchenbeschleuniger (604) abzudecken.

**2.** System nach Anspruch 1, wobei der Applikator (610) ausgelegt ist, um die Apertur (625) in Richtung des Patienten und vom Patienten weg zu bewegen.

**3.** System nach Anspruch 1, wobei das innere Gerüst (601) ausgelegt ist, um den Applikator (610) entlang der Führung (618) in einer kontinuierlichen Weise zu bewegen.

**4.** System nach Anspruch 1, wobei das innere Gerüst (601) ausgelegt ist, um den Applikator (610) in einzelnen Bewegungsschritten entlang der Führung (618) zu bewegen.

**5.** System nach Anspruch 1, außerdem umfassend:

eine Prozessoreinrichtung die programmiert ist, um die Bewegung des äußeren Gerüsts (605) und des inneren Gerüsts (601) zu steuern.

**6.** System nach Anspruch 5, wobei die Prozessoreinrichtung ausgelegt ist, um die Bewegung des äußeren Gerüsts (605) und des inneren Gerüsts (601) zu steuern, um den Protonen- oder Ionenstrahl im Wesentlichen mit der Apertur (625) auszurichten.

**7.** System nach Anspruch 1, wobei die Apertur (635) ausgelegt ist, um den Protonen- oder Ionenstrahl im Wesentlichen zu kollimieren.

**8.** System nach Anspruch 1, wobei der Patienten-Träger (606) relativ zu dem inneren Gerüst (601) und dem äußeren Gerüst (605) bewegbar ist.

**9.** System nach Anspruch 1, wobei der Applikator (610) umfasst:

einen Verlängerungsantrieb (619), um die Apertur (625) in Richtung eines Patienten auf dem Patienten-Träger (606) oder von dem Patienten auf dem Patienten-Träger (606) weg zu bewegen; und
einen Theta-Antrieb (620), um den Applikator (610) entlang unterschiedlicher Positionen der Führung (618) zu bewegen.

**10.** System nach Anspruch 9, wobei die Apertur (625) ausgelegt ist, um eine Querschnittsform des Teilchenstrahls zu ändern.

**11.** System nach Anspruch 1, wobei der Applikator (610) einen Multi-Leaf-Collimator umfasst der ausgelegt ist, um den Partikelstrahl zu dimensionieren und/oder zu formen oder, um es dem Partikelstrahl zu ermöglichen ohne Änderung zu passieren.

**Revendications**

**1.** Système comprenant :

un support de patient (606) destiné à soutenir un patient, le support de patient étant situé au-dessus d'un sol (612) ;
un accélérateur de particules (604) ;
un portique externe (605) qui est déplaçable autour du support de patient (606), l'accélérateur de particules (604) étant installé sur le portique externe (605) et étant configuré pour produire un faisceau de protons ou d'ions, le portique externe (605) étant déplaçable de telle sorte que l'accélérateur de particules se trouve sous le sol (612) ; et

un portique interne (601) comprenant une construction soudée structurelle (617) comprenant un rail porteur (618),

le portique interne (601) comprenant un applicateur (610) installé sur le rail (618), l'applicateur comprenant une ouverture (625) servant à diriger le faisceau de protons ou d'ions vers une cible dans le patient,

le portique interne (601) étant configuré pour déplacer l'applicateur (610) le long du rail (618) en passant par une série de positions autour du patient de manière à positionner le faisceau de protons ou d'ions avec une précision inframillimétrique, et

le portique interne (601) comprenant un couvercle (614) configuré pour rouler le long d'un chemin entre une partie supérieure (621) de la construction soudée structurelle (617) et une partie inférieure (622) de la construction soudée structurelle (617), le couvercle (614) servant à recouvrir un trou dans le sol au-dessus de l'accélérateur de particules (604).

2. Système selon la revendication 1, dans lequel l'applicateur (610) est configuré pour déplacer l'ouverture (625) vers ou loin du patient.

3. Système selon la revendication 1, dans lequel le portique interne (601) est configuré pour déplacer l'applicateur (610) le long du rail (618) d'une manière continue.

4. Système selon la revendication 1, dans lequel le portique interne (601) est configuré pour déplacer l'applicateur (610) le long du rail (618) en mouvements discrets.

5. Système selon la revendication 1, comprenant en outre :

   un dispositif de traitement programmé pour contrôler le mouvement du portique externe (605) et du portique interne (601).

6. Système selon la revendication 5, dans lequel le dispositif de traitement est configuré pour contrôler le mouvement du portique externe (605) et du portique interne (601) afin d'aligner sensiblement le faisceau de protons ou d'ions avec l'ouverture (625).

7. Système selon la revendication 1, dans lequel l'ouverture (625) est configurée pour collimater sensiblement le faisceau de protons ou d'ions.

8. Système selon la revendication 1, dans lequel le support de patient (606) est mobile par rapport au portique interne (601) et au portique externe (605).

9. Système selon la revendication 1, dans lequel l'applicateur (610) comprend :

   une commande d'extension (619) pour déplacer l'ouverture (625) vers ou loin d'un patient sur le support de patient (606) ; et
   une commande thêta (620) pour déplacer l'applicateur (610) le long de différentes positions du rail (618).

10. Système selon la revendication 9, dans lequel l'ouverture (625) est configurée pour changer la forme en coupe transversale du faisceau de particules.

11. Système selon la revendication 1, dans lequel l'applicateur (610) comprend un collimateur multilame configuré pour dimensionner et/ou pour façonner le faisceau de particules ou pour laisser passer le faisceau de particules sans changement.

FIG. 1

EP 2 581 110 B1

10

81

194    34
38    119

12

40

42

70

83

FIG. 2

FIG. 3

FIG. 4

FIG. 5

FIG. 6

FIG. 7

FIG. 8

FIG. 9

FIG. 10

FIG. 11

FIG. 12

EP 2 581 110 B1

FIG. 13

FIG. 14

FIG. 15

FIG. 16

FIG. 17

EP 2 581 110 B1

FIG. 18

EP 2 581 110 B1

EP 2 581 110 B1

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2629333 A1
- US 7014361 B1
- US 20060173294 A1
- US 20070133752 A1
- EP 1419801 A1
- US 18763305 A
- US 59008904 P
- US 94973404 A
- US 59008805 P
- US 76078806 P
- US 46340206 A
- US 85056506 P